(19) 

Europäisches
Patentamt
European
Patent Office
Office européen
des brevets

(11) **EP 2 320 216 A2**

(12) **EUROPEAN PATENT APPLICATION**

(43) Date of publication:
**11.05.2011 Bulletin 2011/19**

(51) Int Cl.:
***G01N 21/65*** (2006.01)

(21) Application number: **10187095.4**

(22) Date of filing: **11.10.2010**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(30) Priority: **12.10.2009 KR 20090096643**

(71) Applicant: **Korea Advanced Institute of Science
and Technology
Daejeon-si 305-701 (KR)**

(72) Inventors:
• **Kim, Bongsoo
Daejeon 305-701 (KR)**

• **Kang, Taejoon
Daejeon 305-701 (KR)**
• **Yoon, Ilsun
Daejeon 305-701 (KR)**
• **Lee, Sang Yup
Daejeon 305-761 (KR)**
• **Yoo, Seung Min
Daejeon 305-701 (KR)**

(74) Representative: **Albrecht, Thomas
Kraus & Weisert
Patent- und Rechtsanwälte
Thomas-Wimmer-Ring 15
80539 München (DE)**

(54) **Detection method of bio-chemical material using surface-enhanced RAMAN scattering**

(57)    Provided is a detection method of a biochemical material using surface-enhanced Raman scattering in order to detect the existence of a biochemical material in a target subject or its content therein, more particularly a detection method of a biochemical material facilitating multiplex detection with high-sensitivity, high-reproducibility, high-reliability, and high-precision owing to multiple hot spots formed on the nanowire surface of a single crystal body by the bond of multiple nanoparticles which are physically separated from each other.

Fig. 1

## Description

## CROSS-REFERENCE(S) TO RELATED APPLICATIONS

The present invention claims priority of Korean Patent Application No. 10-2009-0096643, filed on October 12, 2009, which is incorporated herein by reference.

## BACKGROUND OF THE INVENTION

## Field of the Invention

The present invention relates to a detection method of a biochemical material using surface-enhanced Raman scattering in order to detect the existence of a biochemical material in a target subject or its content therein. More particularly, the present invention relates to a detection method of a biochemical material facilitating multiplex detection with high-sensitivity, high-reproducibility, high-reliability, and high-precision owing to multiple hot spots formed on the nanowire surface of a single crystal body by the bond of multiple nanoparticles which are physically separated from each other.

## Description of Related Art

Studies on genes and proteins have provided a possibility of prediction of a novel biomarker that can predict or diagnose disease. Particularly in the case of cancer, early precise diagnosis is essential for the full recovery and for the prevention of recurrence. So, studies have been actively going on to develop a diagnostic sensor for the early detection of a biomarker based on such antigen-antibody reaction. This kind of sensor is not only applicable for medical diagnosis but also useful in various fields of public health, environment, military, and food industry, etc, triggering continuous active study from different approaches.

There are methods to detect an antigen by using optical equipment in order to detect a small amount of antigen. These methods have been most widely used up to date even though they take high costs, time and efforts. Recently, the following methods have been reported, one of which is the method based on the idea that light wave is different according to the presence of an antigen (Chou et al., (2004) Biosens. Bioelectron. 19, 999-1005); and the other is the method based on the idea that light wave is different according to the changes of nanoparticles having the surface capable of complementary binding with an antigen (Alivisatos et al., (2004) Nat. Biotechnol. 22, 47-52, Nam et al., (2003) Science. 301, 1884-1886; US Patent No. 6,974,669).

SERS (Surface-Enhanced Raman Scattering, referred as SERS hereinafter) is a spectroscopy using the phenomenon that Raman scattering strength is rapidly increased at least $10^6 \sim 10^8$ times when a molecule is absorbed to the nanostructure surface of a metal such as gold and silver, etc. By taking advantage of nano-technique that advances fast these days, this method is expected to be more advanced to a high-sensitive technique facilitating direct detection of one target molecule, and particularly highly expected to be very useful as a medical sensor.

The SERS sensor is technically superior to the electric nano-sensor whose resistance is changed when a molecule is absorbed. The measured value by the resistance sensor is scalar. On the other hand, SERS sensor can give total spectrum of whole vector data, indicating that information obtained from one time measurement by SERS sensor is way huger.

Kneipp and Nie et al first reported that molecules bound on nanoparticles could be measured at a single molecular level by SERS using aggregated nanoparticles. Since then, studies have been made about SERS enhancement using various nano-structures (nanoparticles, nano-shells, nanowires). To use this high sensitive SERS phenomenon for the development of a biosensor, the research team of Mirkin tried and had success in high sensitive DNA analysis using nanoparticles bound with DNA.

Along with high sensitive DNA analysis, approaches have been made to diagnose different diseases including Alzheimer's disease and diabetes early by using the SERS sensor.

SERS phenomenon provides directly the information about molecular vibration or molecular structure which has been provided by Raman spectroscopy. Thus, SERS is a comparatively excellent measurement technique having high-selectivity and high-information, compared with the conventional measurement skills such as laser-induced fluorescence spectroscopy, and as a result it is now recognized as a powerful method for chemical/biological/biochemical analysis with ultra-high sensitivity.

Even with the said advantages above, there are still concerns about SERS because ① the mechanism of SERS has not been fully understood, yet; ② it is difficult to control and synthesize well-defined nano-structure; and ③ reproducibility and reliability are in question considering the changes of enhancement efficiency by light waves and polarization directions which are used for the measurement of spectrum. The said problems have to be solved to apply SERS for the development of a nano-biosensor and commercialization thereof.

To solve those problems, it is essential to understand optical characteristics of well-defined nano-structure and it is further required to study more deeply to control SERS phenomenon using the same.

The research team of Moskovits, Halas, and Van Duyne reported recently that SERS enhancement could be controlled and optimized by using various well-defined nano-structures. Moskovits's team and Yang's team reported that SERS enhancement could be regulated by using the colonies of metal nanowire. In 2006, Moerner's team had success in producing nano-bowtie, the artificial SERS enhanced structure, by using Electron-beam lithography.

The SERS sensor using nanoparticle is most common these days. The SERS basic structure proposed by Binger and Bauer et al is the optical structure composed of metal island film (referred as MIF hereinafter) on the flat metal surface. MIF is composed of random 2-dimensional array metal particles and its maximum size is some nm each. In this structure, the metal particles have various shapes and their arrangement shows probability based random structure, which means it does not show a defined structure. So, it is very difficult for SERS sensor to have reproducibility and accuracy. Regular scattering strength is not guaranteed, either, because of the diversity in metal particle shape.

Hereinbefore, the MIF structure was illustrated as an example of SERS sensor problems. However, it is the general problem that every SERS sensor using metal nanoparticles has. Therefore, there are still problems to solve in well-defined structure which is defined and established by metal particles limited in the size of less than 5 nm, for example, a problem in metal particle shape and difficulty in controlling parameter on the metal surface, etc.

There was another attempt to produce a SERS sensor by using not metal particle but metal nanowire especially Ag nanowire.

Tao et al (Nano. Lett. 2003, 3, 1229) constructed a monolayer comprising a large amount of Ag nanowire on Si wafer by Langmuir-Blodgett method, and then measured SERS using the same. Even though the structure of the sensor and the preparation method proposed by Tao et al took advantage of Ag nanowire and even though the long axis of Ag nanowire comprising the monolayer showed a regular arrangement, reproducibility of SERS result was still in question.

Jeong et al (J. Phys. Chem. B 2004, 108, 12724) synthesized flat array (rafts) of Ag nanowire by using a template. They observed SERS phenomenon by using Ag nanowire rafts arrayed in one direction and confirmed that SERS signals were changed according to the directions of nanowire length and polarization. Jeong et al measured SERS resulted from the interaction between two nanowires and from the difference of polarization direction of laser. However, a huge amount of Ag nanowires were required for SERS because of the flat array structure and solid, high quality Ag nanowire could not be obtained by the preparation method of Ag nanowire. And, it was difficult to control SERS affected by the polarization direction of laser and the interaction between two nanowires.

Aroca et al (Anal. Chem. 2005, 77, 378) had placed a large amount of Ag nanowires synthesized in liquid phase on a glass board, which they used as a SERS board for the measurement of SERS. However, there were a large number of other particles in addition to nanowires and their arrangements were irregular.

Schneider et al (J. Appl. Phys. 2005, 97, 024308) and Lee et al (J. Am. Chem. Soc. 2006, 128, 2200) constructed Ag nanowire array by using a template. They measured SERS with or without eliminating the template. When the template was eliminated, SERS signal was increased.

Proke et al (Appl. Phys. Lett., 2007, 90, 093105) measured SERS after synthesizing ZnO and $Ga_2O_3$ nanowire and then coating with Ag. The structure of the part where SERS occurred was determined by the shape of synthesized ZnO and $Ga_2O_3$ nanowire. $Ga_2O_3$ nanowire was synthesized as a tangled form, while ZnO was synthesized comparatively less tangled. When the tangled $Ga_2O_3$ nanowire was used, higher SERS signal was obtained.

The enhancement of SERS signal reported by Jeong, Proke, Schneider, and Lee via metal particle dimer was the result of those experiments supporting SERS enhancement theories proposed by Brus and Kall saying that SERS was enhanced by hot spot or interstitial field formed in between at least two nanoparticles which are not isolated but neighbored in the distance of 1 - 5 nm. According to the electromagnetic calculation, SERS enhancement was predicted to increase by $10^{12}$ by the hot spot.

Like the optical sensor using metal nanoparticles, the structure of the optical sensor for SERS using nanowire has difficulty in controlling nanowire shape and quality. Physical structure of the prepared nanowire was not clearly defined, either. Considering that the generation of hot spot essential for SERS was not controlled regularly, it was difficult to expect high reproducibility and reliability, indicating that the measurement could not be properly directed. That brought a problem in the development of a sensor. In a nanoparticle aggregate, the location of hot spot and the strength of the spot could be changed by the degree of aggregation. It could bring a big problem in reproducibility and control of the resultant SERS signal.

As explained hereinbefore, the leading research groups of Van Duyne and Halas et al had developed a unique system (nanopattern, nanoshell) and continued their study to control and enhance reproducibility of SERS by using the characteristics of surface plasmon of the system. The development of bio-sensor is on the way using the same. However, the development of SERS optical sensor with precisely controlled hot spot, in which location and structure of each nanowire on the board are regulated, has not been reported except the technique provided by the present invention (Korean Patent No. 0892629), which can be prepared easily with high-purity/high-quality/solid-phase nanowires.

The present inventors have studied to enhance SERS signals of biomolecules such as biological extract, protein, and DNA, and to improve reproducibility, sensitivity and reliability of measurement by using the hybrid structure of nanowire and nanoparticle synthesized in gas-phase whose surface and crystal status are clearly defined, with which the inventors applied for patent.

Hybrid metal-metal nano-structure can play an important role in the development of an efficient bio/chemical sensor using optical signal. This is because they can provide "hot spot", the electromagnetic field focused by LSPR (Localized Surface Plasmon Resonance) coupling, in between nano-structures. Among many optical detection methods, Surface-Enhanced Raman Scattering (referred as SERS hereinafter) is a very fascinating technique useful for the detection of single molecule with high-sensitivity. More importantly, SERS provides excellent selectivity compared with other detection methods because Raman spectrum provides a signal especially against a specific chemical functional group that can be used for the detection of a target material. The said advantage of SERS has made it as a very useful tool for the analysis and identification of biomolecules including DNA and proteins, and other chemical materials.

Noble metal nanoparticles and nanowire can be simply synthesized, so which are two important basic nano-structures usable as SERS-active structures attracting our attention.

Numbers of nano-structures have been proposed up to date which have been established based on such nanoparticles as nanoparticle dimer, aggregate, assembly, and nano-shell. One-dimensional SERS-active structures having both high enhancement and directional response such as nanowire array prepared by using SNOF (Single-Nanowire-On-a-Film), nanowire pair, arranged nanowire bundle, nanowire Langmuir-Blodgett film, and lithography have been also studied.

Hybrid nano-structure, which means two different nano-structures are combined, can be an effective SERS-active structure having the advantages of both nanowire and nanoparticles. Many research groups have reported that nanowire/nanoparticle combined structure demonstrated high-enhancement, polarization-dependency, and remote excitation of Raman signal. Therefore, a single hybrid nano-structure can be used as a tool for the future SERS detection/imaging.

The easy construction of a well-defined hybrid nano-structure and its application for biological study remain as an important work to do for the development of a real SERS-active structure. There have been no reports saying that a biochemical material was detected in nanowire/nanoparticle SERS-active structure.

In the meantime, a technique to detect specific target DNA that is multiplex and sensitive and at the same time facilitates gathering maximum volume of information from a small amount of sample at a low price is urgently requested in the fields of gene profiling, drug screening, and other biomedical fields such as disease diagnosis, etc.

Therefore, a simple, reliable and fast screening method of multiple DNAs has been developed by using different detection methods such as measurement of changes of fluorescence, SPR, electric signal, and mass.

Among many DNA detection methods, fluorescence assay is the general and most preferred technique for multiplex DNA detection. Considering sensitivity at single molecular level, molecular specificity of SERS spectra, effectiveness of excitation source, and no quenching by humidity, oxygen, and other materials, SERS is also regarded as a promising method for the detection of non-labeled multiplex DNA.

Owing to these remarkable advantages, unique SERS sensing platforms have been developed. However, nano-structure dependent SERS signal reproducibility and multiplex DNA detection from a small amount of sample are still tasks to overcome to develop a useful SERS sensor for the realization of multiplex DNA detection.

## SUMMARY OF THE INVENTION

An embodiment of the present invention is directed to providing a detection method of a biochemical material using surface-enhanced Raman scattering(SERS)-active structure composed of nanowire and nanoparticles self-assembled to nanowire.

Another embodiment of the present invention is directed to providing a multiplex detection method of various biochemical materials by single measurement.

Another embodiment of the present invention is directed to providing a detection method of a biochemical material providing high-reproducibility and reliability owing to the well-defined physical structure and well-defined hot spot structure.

Another embodiment of the present invention is directed to providing a detection method of a biochemical material characterized by high-sensitivity with providing SERS spectrum enhanced by the well-defined multiple hot spots.

Hereinafter, the present invention is described in detail.

The detection method of a biochemical material of the present invention is the method to detect the existence or the content of the existing target biochemical material included in a target sample by using SERS (Surface-Enhanced Raman Scattering), which comprises the following steps; (a) contacting the target material with single crystal noble metal nanowire having the first receptor formed thereon and with noble metal nanoparticles having the second receptor formed thereon in order to contact the detection target with the first receptor and with the second receptor, and accordingly to bind the noble metal nanoparticles to the noble metal nanowire with the detection target in the middle; and (b) obtaining SERS spectrum by irradiating polarized laser beam on the noble metal nanowire conjugated with the noble metal nanoparticles, mainly focusing on the nanowire.

The biochemical material, the target subject of detection, includes cell components, genetic materials, carbon compounds, and organic materials involved in metabolism, biosynthesis, transportation, or signal transduction.

Particularly, the biochemical material of the present invention includes high-molecular organic substances, organic metal compounds, peptides, carbohydrates, proteins, protein complexes, lipids, metabolites, antigens, antibodies, enzymes, substrates, amino acids, aptamers, saccharides, nucleic acids, nucleic acid fragments, PNA (Peptide Nucleic Acid), cell extracts and their combinations.

The bond between noble metal nanowire or nanoparticles with the target material for analysis, particularly the bond between the said noble metal nanowire or nanoparticles with the biochemical material included in the target sample, is characteristically the bond between enzyme and substrate, the bond between antigen and antibody, the bond between proteins, complementary bond between DNAs, or the bond between biotin and avidin.

The length of the long axis of the noble metal nanowire is at least 1 μm, and the aspect ratio (long axis length/ short axis length of nanowire) is 5 - 5000. The mean diameter of the noble metal nanoparticles is 5 nm - 20 nm.

The joint density, the number of noble metal nanoparticles bound onto the surface of noble metal nanowire per unit area is equal to the hot spot density, the number of hot spots existed in the surface of noble metal nanowire per unit area.

Particularly, on the noble metal nanowire surface, single noble metal nanoparticle was physically separated and self assembled around the biochemical material, the detection target, by the bond between enzyme-substrate, antigen-antibody, protein-protein, biotin-avidin or the complementary bond between DNAs. So, the number of noble metal nanoparticles bound on the surface of noble metal nanowire per unit area is the number of hot spots on the surface of noble metal nanowire per unit area, which is in another word hot spot density.

The target material for analysis herein includes avidin, and the first receptor and the second receptor include biotin respectively. The noble metal nanoparticles are characterized herein by being self-assembled on the surface of the noble metal nanowire through biotin-avidin-biotin bond that is biotins are combined each other with avidin in the middle. The said avidin herein includes the avidin specifically bound with a biochemical material, the detection target.

Preferably, when the said first receptor and the second receptor include biotin, the said noble metal nanowire or the noble metal nanoparticles are modified with N-(6-(biotinamido)hexyl)-3'-(2'-pyridyldithio)-propionamide, referred as EZ-Link Biotin-HPDP hereinafter) to form biotin on the noble metal nanowire or the noble metal nanoparticles.

The detection target includes target DNA, and the said first receptor includes prove DNA. The said second receptor includes Raman dye conjugated reporter DNA. The noble metal nanoparticles are characteristically self-assembled on the surface of the noble metal nanowire via the complementary bond between the target DNA and the probe DNA and the complementary bond between the target DNA and the reporter DNA.

Characteristically, the target DNA includes pathogenic DNA or pathogenic DNA extracted and isolated from a living subject infected with the pathogen.

More precisely, the target DNA is bound complementarily with the probe DNA formed on the surface of the noble metal nanowire and at the same time bound complementarily with the reporter DNA formed on the surface of noble metal nanoparticle. The Raman dye that is conjugated on the reporter DNA to increase signal sensitivity is preferably selected by considering the wavelength of laser. For an example, in the case of 633 nm He-Ne laser, Cy5 was used as Raman dye.

The detection method of a biochemical material of the present invention characteristically facilitates the detection of different biochemical materials from each noble metal nanowire by contacting the detection target with at least two of single crystal noble metal nanowires on which different first receptors are formed with being physically separated each other and nanoparticles on which different second receptors are formed.

Characteristically, the detection target is contacted with at least two single crystal noble metal nanowires having different probe DNAs formed on their surfaces and noble metal nanoparticles having different reporter DNAs formed thereon, and as a result different target DNAs are detected from each noble metal nanowires.

Characteristically in this invention, at least N (natural number, N>1) of single crystal noble metal nanowires on which different probe DNAs are formed with being physically separated each other and single noble metal nanoparticles on which Raman dye conjugated reporter DNAs are formed are contacted with the detection target containing 1 - N numbers of target DNAs and as a result different target DNAs are detected from each noble metal nanowire.

In the multiplex detection herein, at least one of the noble metal nanowires are characteristically identified by location addressing on the board.

The said noble metal nanowire is Au, Ag, Pt or Pd nanowire, and the said noble metal nanoparticle is also Au, Ag, Pt or Pd nanoparticle, which is the same as the noble mental nanowire.

The said surface enhanced Raman scattering is characteristically generated by irradiation of polarized laser beam having the noble metal nanowire as a focus in the direction of long axis of the single noble metal nanowire. The angle θ made by the long axis of the noble metal nanowire and the polarization direction of the laser beam is 30 - 150° or 210 - 330°.

**BRIEF DESCRIPTION OF THE DRAWINGS**

Figure 1 is an example of the detection method of a biochemical material of the present invention.

Figure 2 is another example of the detection method of a biochemical material of the present invention.

Figure 3 is an example of the multiplex detection method of biochemical materials of the present invention.

Figure 4 is a set of SEM photographs. Figure 4(a) is a SEM photograph of Au nanowire on which Au nanoparticles are self-assembled via biotin-avidin-biotin bond. Figure 4(b) is a SEM photograph of avidin-free Au nanowire.

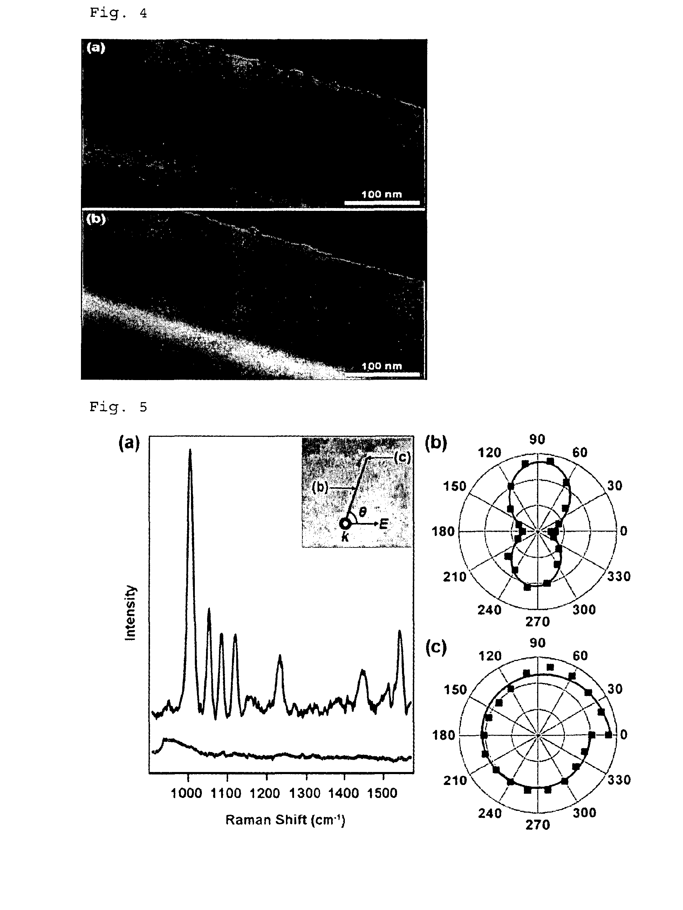

Figure 5 shows the SERS spectrum of Au nanowire on which Au nanoparticles are self-assembled via biotin-avidin-biotin bond (Figure 5(a), blue), the SERS spectrum of avidin-free Au nanowire (Figure 5(a), purple), 1120 cm$^{-1}$ Raman band integrated value according to θ, the angle made by the long axis direction of nanowire measured in (b) area of the nanowire shown in Figure 5(a) and the polarization direction of laser beam (Figure 5(b)), and 1120 cm$^{-1}$ Raman band integrated value according to θ, the angle made by the long axis direction of nanowire measured in (c) area of the nanowire shown in Figure 5(a) and the polarization direction of laser beam (Figure 5(c)).

Figure 6 shows the SERS spectrum (Figure 6(a)) according to the concentration of avidin added to PBS solution. in which biotin conjugated Au nanoparticle and biotin conjugated Au nanowire are dispersed, the number of Au nanoparticles (Figure 6(b), blue) attached on Au nanowire in the area of 1x10$^4$ nm$^2$ according to the avidin concentration, and the number of Au nanoparticles (Figure 6, purple) attached on Au nanowire in the area of 1x10$^4$ nM$^2$ according to the non-specific binding protein concentration.

Figure 7 comprises a schematic diagram (Figure 7(a)) illustrating the detection method of DNA, SERS spectra (Figure 7(b)) resulted from the contact with specific DNA (blue) and non specific DNA (purple), and a set of SEM photographs (Figure 7(c)) resulted from the contact with specific DNA and non specific DNA.

Figure 8 comprises a schematic diagram (Figure 8(a)) illustrating the detection method using multiplex platform containing two nanowires having different probe DNAs, and SERS spectra (Figure 8(b)) resulted from multiplex detection.

Figure 9 comprises a schematic diagram (Figure 9(a)) illustrating the detection method using multiplex platform containing 4 kinds of nanowires having different probe DNAs, SERS spectra (Figure 9(b)) resulted from multiplex detection, another SERS spectra (Figure 9(c)) resulted from the detection of target DNA, and another SERS spectra (Figure 9(d)) resulted from serial multiplex detection.

Figure 10 is a set of graphs, one of which illustrates the changes of SERS spectrum over the mol of the detection target DNA (Figure 10(a)) and the other illustrates the relation between mol of the detection target DNA and the strength of SERS spectrum (Figure 10(b)).

Figure 11 is a set of graphs illustrating the results of SERS detection of multiplex pathogenic bacteria DNA (reference bacteria DNA) by using Au nanowire-Au nanoparticle structure

Figure 12 is a graph illustrating the results of SERS detection of clinic bacteria DNA by using Au nanowire-Au nanoparticle structure.

[Detailed Description of Main Elements]

100: noble metal nanowire 200: noble metal nanoparticle
110: first receptor 210: second receptor
300: biochemical material

DESCRIPTION OF SPECIFIC EMBODIMENTS

The advantages, features and aspects of the invention will become apparent from the following description of the embodiments with reference to the accompanying drawings, which is set forth hereinafter.

The detection method of a biochemical material of the present invention is described in detail with the attached figures. The following figures are presented as examples to deliver the purpose and spirit of the present invention to those in the art. Therefore, the present invention is not limited to the presented figures and can be embodied in different ways. The same reference number in this description indicated the same component.

Unless stated otherwise, the technical terms and the scientific terms used in this description can be understood as the general meaning by those in the art. Explains that might mislead the idea about the functions and structures of the present invention are excluded in the following embodiments and attached figures.

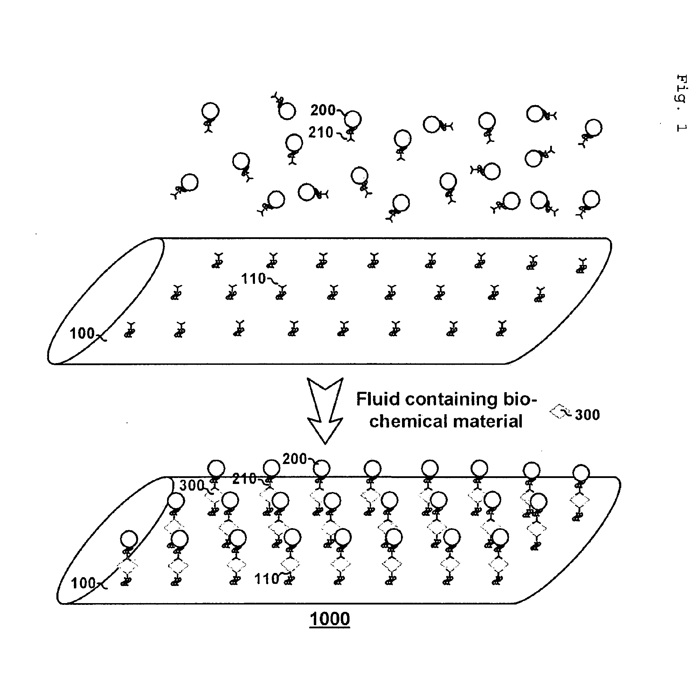

Figure 1 is an example of the detection method of a biochemical material of the present invention. Particularly in this method, the first receptor (110) was formed on the surface of the noble metal single crystal nanowire (100); the second receptor (210) was formed on the surface of the noble metal nanoparticle (200); and the noble metal single crystal nanowire (100) and the noble metal nanoparticle (200) were contacted with the detection target containing the detection target biochemical material (300) so that the noble metal nanoparticle (200) was bound with the noble metal nanowire (100) with having the biochemical material (300) in between them.

Particularly, the first receptor (110) bound on the surface of the noble metal single crystal nanowire (100) and the second receptor (210) bound on the surface of the noble metal nanoparticle (200) were respectively bound with the target biochemical material included in the detection target, so that the noble metal nanoparticles (200) were self-assembled on the surface of the noble metal nanowire (100) and accordingly the detection target biochemical material (300) was fixed in between the noble metal nanowire (100) and the noble metal nanoparticle (200).

To maximize Raman signal strength of a molecule (biochemical material) attached onto the nanostructure, a biochemical material has to be located in between arranged nanostructures like noble metal nanowires and noble metal nanoparticles of the present invention. Such arranged nanostructure is characteristically SERS-active nanostructure turned on by the bond of a biochemical material with nanowire and nanoparticle.

The said noble metal single crystal nanowire (100) had the aspect ratio (long axis length of nanowire/short axis diameter) of 5 - 5000. The length of the long axis was at least 1 $\mu$m The noble metal single crystal nanowire (100) herein was characteristically nanowire aggregate in which two or more nanowires were physically aggregated; nanowire conjugate in which two or more nanowires were physically conjugated; or free standing single nanowire, that was not a complex in which nanowire was conjugated with a homologous material or a heterologous material.

The surface enhanced Raman scattering spectrum could be obtained by irradiating polarized laser beam on the noble metal nanowire (100) combined with the noble metal nanoparticle (200) with focusing the beam on the noble metal nanowire (100). At this time, the SERS spectrum obtained by irradiating laser beam was the result of enhancement caused by local electromagnetic field formed by the single noble metal nanowire and multiple noble metal nanoparticles.

The mean diameter of the noble metal nanoparticle (200) was 5 nm - 20 nm. This was the preferred size in order for the noble metal nanoparticle (200) to be combined with the noble metal nanowire (100) to form hot spot, the focused electromagnetic field area by LSPR (Localized Surface Plasmon Resonance) coupling, and at the same time to form high density hot spot on the noble metal nanowire (100).

As shown in Figure 1, the detection method of the present invention according to the description in Figure 1 is characterized by the followings: noble metal nanoparticles were self-assembled on the noble metal nanowire by the bond of the first receptor-detection target material attached on the surface of the noble metal nanowire and the second receptor attached on the surface of the noble metal nanoparticle; and the noble metal nanowire was high purity, high crystalline single crystal body, and a single nanowire with flat surface having even thickness and big aspect ratio (surface is hardly rough), and thus noble metal nanoparticles were evenly bound on the surface of the noble metal nanowire and even hot spots were formed by such mono noble metal nanoparticle and the noble metal nanowire with demonstrating very high joint density of the noble metal nanoparticles.

For an example, in the case that the first receptor and the second receptor include biotin respectively and the detection target material contains avidin, the joint density that is the number of noble metal nanoparticles bound on the noble metal nanowire per unit area was 1500 - 3500 (number/$\mu$m$^2$). According to the description herein, the joint density, that is the number of noble metal nanoparticles bound on the surface of the noble metal nanowire per unit area, was consistent with the hot spot density which is the number of hot spots located in the unit area on the surface of the noble metal nanowire. So, the hot spot density was also 1500 - 3500 (numbers / $\mu$m$^2$) .

The noble metal nanowire of the present invention was characteristically Au, Ag, Pt or Pd nanowire, and the noble metal nanoparticle of the present invention was characteristically Au, Ag, Pt or Pd nanoparticle, and it was more preferred herein that the said noble metal nanowire was the same noble metal material as the noble metal nanoparticle.

Particularly, the noble metal nanowire on which the first receptor was formed was located on the SERS inactive board. And the noble metal nanowire was contacted with the detection target in a liquid phase.

Dispersion of the noble metal nanoparticles on which the second receptor was formed was applied on the noble metal nanowire contacted with the detection target or the noble metal nanowire contacted with the detection target was dipped in dispersion of the noble metal nanoparticles on which the second receptor was formed.

Particularly, the SERS inactive board with the noble metal nanowire on which the first receptor was formed was dipped in dispersion of the noble metal nanoparticles on which the second receptor was formed, during which the detection target was added to the dispersion to contact the detection target with the noble metal nanowire and noble metal nanoparticles.

Particularly, the SERS inactive board with the noble metal nanowire on which the first receptor was formed was dipped in liquid. Then, the detection target and the noble metal nanoparticle dispersion were added to the liquid in which the noble metal nanowire was dipped (either adding the detection target before adding the noble metal nanoparticle dispersion or vise versa) to contact the detection target with the noble metal nanowire and the noble metal nanoparticles.

At this time, the contact of the detection target with the noble metal nanowire and the noble metal nanoparticle could be performed in the device for fluid transportation including micro fluidic capillary tube.

The liquid herein (noble metal nanowire containing solution, noble metal nanoparticle dispersion or detection target containing solution) was not supposed to be reacted chemically with the biochemical material and the receptors formed on the noble metal nanowire (100) and the noble metal nanoparticle (200), but facilitated the dispersion of the noble metal nanoparticle (200) and movement of the biochemical material and the noble metal nanoparticle.

SERS spectrum obtained after contacting with the detection target could be analyzed by using reference SERS spectrum obtained with the same liquid under the same conditions except that the biochemical material was not included.

After combining the noble metal nanoparticle with the noble metal nanowire through the detection target material, SERS spectrum was preferably obtained by irradiating polarized laser beam after separating or washing the noble metal nanowire conjugated with multiple noble metal nanoparticles from the liquid.

At this time, a protein anti-absorption layer could be added on the surface of the noble metal nanowire (100) on which the noble metal nanoparticles (200) were self-assembled in order to prevent protein absorption resulted from non-specific binging. As an example, the anti-absorption layer could be formed by using a hydrophilic molecule such as glycol compound.

The biochemical material, the detection target material included in the detection target was specifically bound to the first receptor formed on the noble metal nanowire (100) and at the same time specifically bound to the second receptor (210) formed on the noble metal nanoparticle (200), suggesting that this material had the binding capacity with at least two subjects.

The binding of the biochemical material and the receptor (110 or 210) was performed by ionic bond, covalent bond, hydrogen bond, coordinate bond or non-covalent bond, and more particularly by enzyme-substrate binding, antigen-antibody binding, protein-protein binding, complementary binding between DNAs, or biotin-avidin binding.

Characteristically in this invention, the first receptor and the second receptor included biotin respectively and the biochemical material herein included avidin.

Characteristically in this invention, one of those receptors, the first receptor and the second receptor, was Raman Dye conjugated DNA. More precisely, the biochemical material included target DNA, the first receptor included prove DNA, and the second receptor formed on the noble metal nanoparticle included Raman dye conjugated reporter DNA.

Characteristically in this invention, the biochemical material, the detection target, was DNA. DNA concentration (M), particularly at the concentration of $10^{-11}$ - $10^{-8}$, was linearly in proportion with the strength of SERS spectrum of step b).

As described hereinbefore, the detection method of a biochemical material of the present invention was characterized by the bond between the noble metal nanowire (100) and the noble metal nanoparticles (200) through the biochemical material (300), the target of detection. And the noble metal nanoparticles (200) were self-assembled on the noble metal nanowire (100) via the biochemical material (300).

Therefore, the presence or absence of the biochemical material (300) determined the presence or absence of hot spot via the bond between the noble metal nanoparticle (200) and the noble metal nanowire (100), and the concentration of the biochemical material (300) determined the surface density of the hot spot.

So, the biochemical material (300), the detection target, was fixed in the binding area of the noble metal nanoparticles (200) and the noble metal nanowire (100), and the surface density of hot spot was determined by the concentration of the biochemical material (300). Thus, SERS spectrum was obtained by irradiating polarized laser beam on the center of the noble metal nanowire (100), followed by the analysis of the presence/absence of the biochemical material, the detection target, the type of the biochemical material, and the concentration of the biochemical material.

The first receptor formed on the noble metal nanowire (100) could be the same as or different receptor from the receptor (210) formed on the noble metal nanoparticle (200), according to the binding capacity of the biochemical material (300).

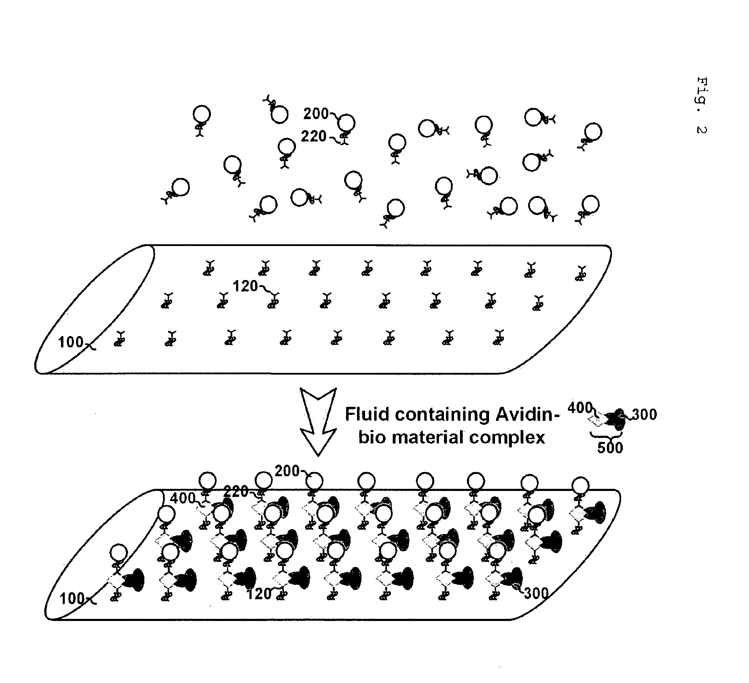

Characteristically, as shown in Figure 2, the first receptor (120) formed on the noble metal nanowire (100) and the second receptor (220) formed on the noble metal nanoparticle (200) included biotin respectively, and the biochemical material, the detection target, was characteristically the biochemical material complex (500) composed of avidin (400) and the biochemical material (300).

Avidin (400) was bound with biotin on the first receptor (120) and the same avidin (400) was combined with biotin on the second receptor (220). As a result, the noble metal nanoparticle (200) was bound with the noble metal nanowire (100) having the biochemical material complex (500) in the middle to generate hot spot.

As described hereinbefore, the presence/absence of the biochemical material (500) determined the presence/absence of hot spot on the noble metal nanowire. And also, the concentration of the biochemical material determined the number of hot spots formed on the surface of the noble metal nanowire.

Figure 2 is another example of the detection method of a biochemical material of the present invention described based on Figure 1. Herein, the first receptor (120) and the second receptor (220) included biotin respectively. The detection target was avidin (400) or the biochemical material complex (500) which was avidin conjugated biochemical material (300).

The noble metal nanoparticles (200) formed hot spot around the biochemical material complex (500) via biotin-avidin-biotin conjugate generated by binding biotin formed on single crystal single noble metal nanowire (100) with biotin formed on the noble metal nanoparticle (200) with avidin in between. SERS spectrum was obtained by irradiating polarized laser beam on the center of the noble metal nanowire (100), followed by the analysis of the presence/absence of the biochemical material, the detection target, the type of the biochemical material, or the concentration of the biochemical material.

At this time, the nanostructure (and noble metal nanowire) in which the noble metal nanoparticle (200) was bound with the noble metal nanowire (100) with the biochemical material complex (500) in the middle could be located on the board for physical support, and could be fixed on the board by van der Waals force of the nanostructure (and noble metal nanowire) and the board or gravity.

When the nanostructure was irradiated with laser beam, SERS signals of the biochemical material (500) which had been enhanced by hot spot of the nanostructure could be obtained.

Biotin attached on the surface of the noble metal nanoparticle (or noble metal nanowire) could be formed by surface modification of the noble metal nanoparticle (or noble metal nanowire). For an example, it was more preferred that the noble metal nanoparticle (or noble metal nanowire) was modified with EZ-Link Biotin-HPDP.

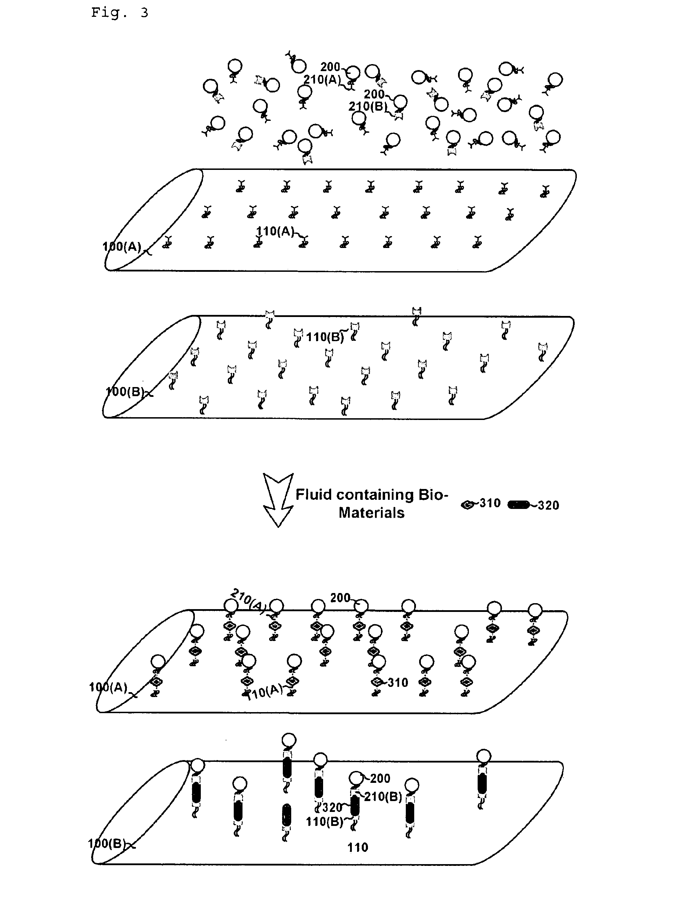

Figure 3 illustrates an example of the multiplex detection of different biochemical materials (310, 320) from different noble metal nanowires located on SERS board which are physically separated from each other. In this Figure, the detection target was contacted with two or more single crystal noble metal nanowires (100(A), 100(B)) on which different first receptors (110(A), 110(B)) were formed but separated physically and two or more noble metal nanoparticles (200(A), 200(B)) on which different second receptors (210(A), 210(B)) were formed for the multiplex detection.

As shown in Figure 3, for the multiplex detection, the first biochemical material (310) was detected by using the noble metal nanowire on which the first receptor (110(A)) specifically binding with the first biochemical material (310) was formed and the noble metal nanoparticle on which the second receptor (210(A)) specifically binding with the first biochemical material (310) was formed. Then, the second biochemical material was detected by using the noble metal nanowire on which the first receptor (110(B)) specifically binding with the second biochemical material (320) was formed and the noble metal nanoparticle on which the second receptor (210(B)) specifically binding with the second biochemical material (320) was formed. Figure 3 illustrates the example of independent detection of two kinds of biochemical materials. To detect N (N is a natural number bigger than 1, N>1) different biochemical materials by a single test, multiplex detection with N biochemical materials could be performed by using N noble metal nanowires on which the first receptors specifically binding with each biochemical material on SERS inactive board were formed and N kinds (noble metal nanoparticles having the same receptors formed thereon are regarded as the same kind) of noble metal nanoparticles on which the second receptors specifically binding with each biochemical material were formed.

More importantly, for the multiplex detection of non labeled DNA, at least two first receptors (110(A), 110(B)) physically separated from each other on the single crystal noble metal nanowires were supposed to be different probe DNAs, and at least two second receptors (210(A), 210(B)) on the noble metal nanoparticles were supposed to be different reporter DNAs. The detection targets include different target DNAs, by which different target DNA could be detected from each noble metal nanowire.

_Detection of non-labeled biochemical material by using noble metal nanowire-noble metal nanoparticle binding_

The following example presents the first result of the detection of a non-labeled biochemical material using single crystal single noble metal nanowire-noble metal nanoparticle binding. For the measurement, Au nanowire and Au nanoparticle were used as SERS active metals, but the present invention is not limited thereto.

The noble metal nanowire of the present invention was preferably prepared by the method described in Korean Patent Application Nos. 10-2007-0065030, 10-2008-0036360, and 10-2009-0035533 applied by the present inventors.

Particularly, the noble metal nanowire was characteristically the noble metal single crystal nanowire prepared on the single crystal board by heat-treating the precursor containing noble metal oxide, noble metal material or noble metal halide located in the front part of the reactor and semiconductor or nonconductor single crystal board located in the rear part of the reactor in the presence of inactive gas.

According to the preparation method of noble metal single crystal nanowire, noble metal nanowire was formed on the single crystal board by using noble metal oxide, noble metal material or noble metal halide as a precursor without using a catalyst. The noble metal single crystal nanowire was formed through gas phase transportation pathway, so that the nanowire showed high-purity and high-quality without any impurities.

According to this method, the temperature of each the front part and the rear part of the reactor could be regulated. In addition, nucleation force, growth force, nucleation rate and growth rate of the metal material were lastly regulated on the single crystal board by controlling inactive gas flow and inner tube pressure of heat-treatment tube used for the heat treatment. Therefore, the size of the noble metal single crystal nanowire and the on-board density could be controlled. So, reproducible, flawless, high quality, and high crystalline noble metal single crystal nanowire could be prepared.

The noble metal single crystal nanowire prepared by the said gas phase transport method and equipped in the biosensor of the present invention was characteristically flawless single crystal body that had no twin or plane fault such as stacking fault, etc. Aspect ratio (nanowire long axis length/short axis diameter) was 5 -- 5000. The length of long axis was at least 1 $\mu$m. The noble metal single crystal nanowire of the present invention was characteristically nanowire aggregate in which two or more nanowires were physically aggregated; nanowire conjugate in which two or more nanowires were physically conjugated; or free standing single nanowire but not a complex comprising nanowire combined with homologous or heterologous material. The nanowire was a solid phase nanowire having even thickness and smooth surface at atomic level.

Au nanowire

Au single crystal nanowire was synthesized in a reactor by using gas phase transport method.

The reactor was divided into the front part and the rear part and was independently equipped with heating element and temperature controller. The tube in the reactor was 1 inch in diameter and 60 cm in size which was made of quartz.

The boat shaped vessel made of high purity alumina containing 0.05 g of the precursor $Au_2O_3$ (Sigma-Aldrich, 334057) was located in the center of the front part of the reactor. The sapphire single crystal board with the surface of (0001) was located in the center of the rear part of the reactor. Argon gas flew from the front part of the reactor through the rear part. A vacuum pump was equipped in the rear part of the reactor. The pressure in the quartz tube was maintained as 15 torr by using the vacuum pump. 500 sccm Ar was flowing by using MFC (Mass Flow Controller).

While maintaining the temperature of the front part of the reactor (alumina boat containing the precursor) at 1100°C and the temperature of the rear part at 900°C, Au single crystal nanowire was prepared by heat-treatment for 30 minutes.

The diameter of the short axis of Au single crystal nanowire was 50 - 150 nm and the length of the long axis was at least 50 $\mu$m. The prepared Au single crystal nanowire had smooth surface at atomic level.

Detection of avidin

Biotin was formed on the surface of the prepared Au single crystal nanowire by surface modification. Particularly, the prepared Au single crystal nanowire was dipped in 1 mL of 0.4 mM EZ-Link Biotin-HPDP (Pierce, 21341) Dimethylformamide (DMF) solution, leading to surface modification in order to form biotin. After washing with DMF solvent, the nanowire was stored in 1 mL of PBS (Phosphate Buffered Saline) solution. Likewise, Au nanoparticles (Sigma-Aldrich, G1527) having the mean diameter of 10 nm were dipped in 2 $\mu$l of 0.4 mM EZ-Link Biotin-HPDP (Pierce, 21341) DMF solution, leading to surface modification to form biotin.

5 mL of avidin (Sigma-Aldrich, A9275) PBS solution (conc: M) was added to Au nanowire having biotin, followed by washing with PBS. Then, 1 mL of Au nanoparticle solution containing 1 mL of biotin was added thereto.

The above processes were performed at room temperature for 180 minutes. To eliminate non-specifically bound Au nanoparticles, the nanowire was washed with PBS. Then, Au nanowire was separated and tested for physical and optical properties.

Figure 4(a) is a SEM (Scanning Electron Microscopy) photograph of Au nanowire obtained after adding Au nanoparticles and avidin to Au nanowire. Biotin formed on Au nanowire and biotin formed on Au nanoparticle were bound each other having avidin in the middle, producing biotin-avidin-biotin bond. Au nanoparticles were self-assembled with avidin in the middle on the single Au nanowire.

Au nanoparticles self--assembled on Au nanowire could be clearly identified. Au nanoparticles did not form an aggregate and were rather physically separated each other, that is single nanoparticles were self-assembled on Au nanowire. Moreover, Au nanoparticles were self-assembled with even and high density on the surface of Au nanowire.

Noble metal nanoparticles were self-assembled on the single noble metal nanowire via a biochemical material and thus the biochemical material was located in the binding region, so called hot spot, between the noble metal nanoparticle and the noble metal nanowire. At this time, Raman signal of the biochemical material was significantly increased, by which the biochemical material could be easily detected.

The nanowire-nanoparticle structure mediated by a biochemical material, the detection target, can be used as a sensor for detecting a biochemical material. Therefore, this structure can be highly applicable for the multiplex detection that facilitates the simultaneous detection of different biochemical materials by modifying the surfaces of different nanowires with various materials and then by placing them on one board.

In the meantime, as shown in Figure 4(b), when Au nanoparticles were forced to be self-assembled on nanowire only with PBS without adding avidin to the dispersion of Au nanoparticle and Au nanowire, Au nanoparticles were not self-assembled on Au nanowire.

Au nanowire obtained in the presence of avidin and Au nanowire (comparative example) obtained in the absence of avidin were located on the Si single crystal board, followed by measurement of SERS spectrum. To measure SERS spectrum, 633 nm laser beam was irradiated on the noble metal nanowire, followed by detection with confocal Raman spectrometer. The strength of laser beam was 0.5 mW and the measurement was continued for 60 seconds.

Figure 5(a) is SERS spectrum of Au nanowire on which Au nanoparticles were self-assembled via biotin-avidin bond (referred as nanostructure SERS spectrum hereinafter). Once hybrid nanostructure was formed in the presence of avidin, a very strong SERS signal was observed (Figure 5a, blue spectrum). Characteristic spectrum could not be obtained from Au nanowire contacted with Au nanoparticles conjugated with biotin in the absence of avidin, except regular Si signal (Figure 5a, wine color spectrum).

This result is consistent with that of optical observation shown in Figure 4. That is, hot spot has been formed according to the binding of noble metal nanowire and noble metal nanoparticle. Very strong SERS spectrum could be obtained by this hot spot. From the single noble metal nanowire on which hot spot was not generated, only Si spectrum was obtained because the surface plasmon excitation was very small.

Enhancement rate of SERS (EF) by the formation of Au nanowire-Au nanoparticle structure was calculated by the following mathematical formula 1.

$$\text{(Mathematical Formula 1)}$$

$$EF = (I_{sers} \times N_{bulk}) / (I_{bulk} \times N_{sers})$$

$I_{sers}$ and $I_{bulk}$ indicate the size of same peak (strength of each specific band) in nanostructure SERS spectrum and in solid phase EZ-Link Biotin HPDP bulk spectrum, and $N_{bulk}$ indicates the number of molecules involved in bulk spectrum, and $N_{sers}$ indicates the number of molecules involved in nanostructure SERS spectrum.

As shown in Figure 5 (a) , in the nanostructure SERS spectrum presented as blue, 1005 cm$^{-1}$ band was the biggest in the spectrum, so that this band was selected as $I_{sers}$ and $I_{bulk}$. $N_{bulk}$ was determined based on the solid phase EZ-Link Biotin HPDP (0.5 g/CM$^3$) Raman spectrum and Raman system focus volume (0.5 femtoL).

When $N_{sers}$ was calculated in the hot spot region, it was assumed that EZ-Link Biotin HPDP was absorbed into the monolayer at the unit area per molecule of 0.4 nm$^2$ on Au nanowire and Au nanoparticle. So, only 3 nm molecules were selected. The number of Au nanoparticles irradiated with laser beam (the number of hot spots involved in SERS signal enhancement) was about 250, as shown in the SEM photograph of Figure 4 (a) , suggesting that $N_{trap}$ was about 2.2 x 10$^5$.

In Figure 5(a), molecules did not show absorption band around 633 nm in nanostructure SERS spectrum, indicating that resonance Raman effect was excluded. It was expected that SERS effect could be much enhanced when a probe molecule having resonance effect was used and laser wave length was optimized.

As shown in Figure 5 (b) and 5 (c) , the strength of nanostructure SERS spectrum varies from the polarization property of laser beam irradiated on the nanostructure of Au nanowire on which Au nanoparticles are self-assembled by biotin-avidin bond and from the irradiation location. Figure 5(b) illustrates the irradiation of laser beam on the center of the long axis of Au nanowire on which Au nanoparticles are self-assembied (location(b) on nanowire shown in Figure 5(a)). Figure 5(c) illustrates the irradiation of laser beam on the long axis tip of Au nanowire on which Au nanoparticles are self-assembled (location(c) on nanowire shown in Figure 5(a)). Polar line to integrated value of 1120 cm$^{-1}$ Raman band according to θ, the angle made by the long axis direction of nanowire and the polarization direction of laser beam is presented.

When the polarization direction of laser beam and the long axis direction of nanowire were crossed at right angle, the strength of SERS was the highest. When the polarization direction of laser beam and the long axis direction of nanowire were paralleled, the strength became the lowest. Such polarization dependency was fitted by $\cos^2\theta$ function correcting the reduction by photoreaction.

On the contrary, SERS signal polarization anisotropy on the tip of Au nanowire was way smaller than that of the center area. Polar line on the tip was fitted by exponential decline function.

The above results can be explained by the difference of surface plasmon excitations between the tip and the center of Au nanowire on which Au nanoparticles are self-assembled. When single nanoparticle was conjugated in the center of nanowire (in the center of the long axis direction of nanowire), strong polarization anisotropy is observed. On the noble metal nanowire of the present invention, multiple noble metal nanoparticles physically separated from each other are self-assembled. Therefore, the observed polarization dependency is explained by the set of electromagnetic fields generated in multiple hot spots.

In the meantime, on the tip of nanowire, the partial structure is like a hemisphere covering a cylinder. In a big sphere covered by small nanoparticles evenly, polarization anisotropy disappears. So, it is preferred to irradiate laser beam on the center of the long axis direction of nanowire to detect a biochemical material with high sensitivity, reliability, and reproducibility.

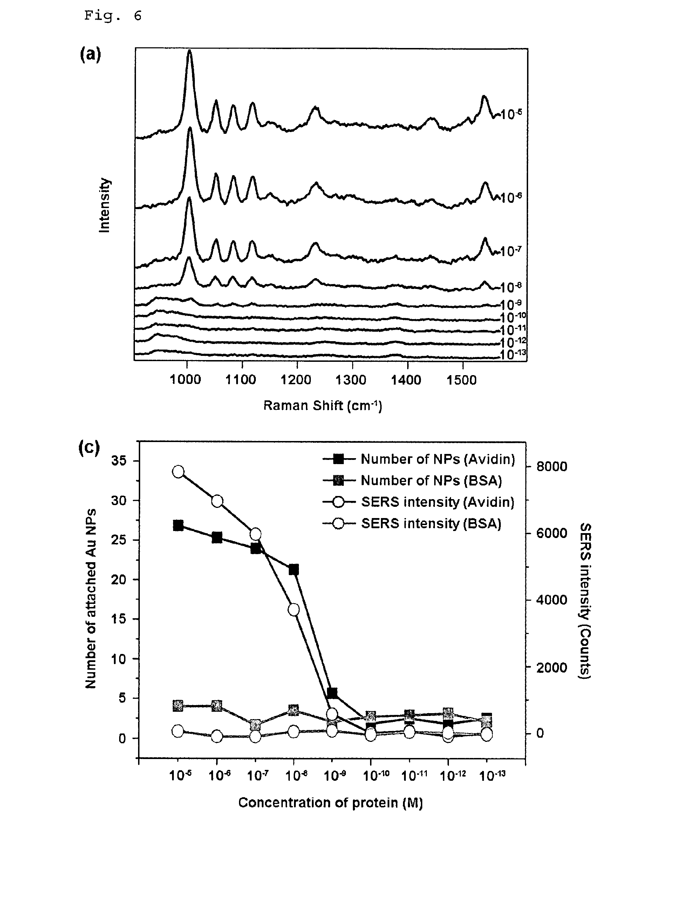

Figure 6(a) illustrates the changes of nanostructure SERS spectrum over the concentration of avidin added to PBS solution in which Au nanowire conjugated with biotin and Au nanoparticle conjugated with biotin were dispersed.

The concentration of avidin in PBS in which Au nanoparticle and Au nanowire were dispersed was preferably regulated to be $10^{-13}$ M - $10^{-5}$ M. Then, Au nanowire was recovered and SERS spectrum was obtained.

As shown in Figure 6(a), when avidin concentration was higher than $10^{-8}$ M, very clear SERS spectrum was observed. SERS strength became reduced at the avidin concentration of $10^{-9}$ M, which was because the number of Au nanoparticles conjugated on Au nanowire was reduced. Raman signal was hardly detected at the concentration of under $10^{-10}$ M. Biotin-avidin binding was an irreversible reaction showing very big thermodynamic affinity constant. Each spectrum shown in Figure 6(a) is the result obtained from different samples respectively. This result confirms that nanostructure SERS signal from Au nanowire on which Au nanoparticles are self-assembled by biotin-avidin bond can be reproduced by hot spot formed evenly on the surface of Au nanowire.

Reproducibility and stability of SERS signal of SERS active structure are very important properties for a good sensor. Au nanowire has an even surface, and Au nanoparticle can also be distributed on the nanowire without aggregation. So, the nanostructure of the present invention shows reliable reproducibility of SERS signals.

To confirm whether or not SERS enhancement was dependent on the specific recognition of avidin, Bovine Serum Albumin (referred as BSA hereinafter), the non-specific binding protein was added to PBS solution in which biotin conjugated Au nanoparticle and biotin conjugated Au nanowire were dispersed in the absence of avidin.

Figure 6(b) shows the number of Au nanoparticles attached on Au nanowire at the concentrations of BSA and avidin based on the SEM photograph. At this time, the observed area of Au nanowire was $1 \times 10^4$ nm$^2$.

As shown in the graph of Figure 6(b), self-assembled Au nanoparticle-Au nanowire structure was not observed even when BSA concentration was increased in PBS solution in which biotin conjugated Au nanoparticle and biotin conjugated Au nanowire were dispersed. This result indicates that the formation of noble metal nanoparticle-noble metal nanowire nanostructure is highly selective for the biotin-avidin binding.

As shown in the graph based on the experiment with avidin of Figure 6(b), the number of hot spots resulted from the self-assembly of noble metal nanoparticles is controlled by avidin concentration.

Detection of target DNA

Hereinafter, an example of DNA detection under similar conditions of Au nanowire and Au nanoparticle to those used for avidin detection is described in detail.

Table 1 is the summary of sequences of probe DNA (Efm003-20, Sau001-20, Sma103-20, Vvul02-20), target DNA (T1 ~ T6), and reporter DNA (R1 ~ R3) used for DNA detection.

As shown in Table 1, Cy5 or TAMRA Raman dye was conjugated to the 5'-termini of reporter DNA to increase sensitivity.

(Table 1) DNA sequences (Genotech, Daejeon, Korea)

| Name | Length (-mer) | Sequence (5'→3') |
|---|---|---|
| Efm003-20 | 20 | SH-(CH$_2$)$_6$-ACATAGCACATTCGAGGTAG |
| Sau001-20 | 20 | SH-(CH$_2$)$_6$-CAAAGGACGACATTAGACGA |
| Smal03-20 | 20 | SH- (CH$_2$)$_6$-GCCATTCCAGTGARGACGAG |
| Vvul02-20 | 20 | SH- (CH$_2$)$_6$-GTAGTTGACGATGCATGTTC |
| T1 | 40 | agtaccgtgagggaaaggcgctacctcgaatgtgctatgt |
| T2 | 40 | tgttacgattgtgtgaatactcgtctaatgtcgtcctttg |
| T3 | 35 | ttccctcacggtactctacctcgaatgtgctatgt |
| T4 | 35 | ttccctcacggtacttcgtctaatgtcgtcctttg |
| T5 | 35 | ttccctcacggtactctcgtcttcactggaatggc |
| T6 | 35 | ttccctcacggtactgaacatgcatcgtcaactac |
| R1 | 20 | Cy5-CGCCTTTCCCTCACGGTACT- (CH$_2$)$_3$-SH |
| R2 | 20 | TAMRA-gtattcacacaatcgtaaca- (CH$_2$)$_3$-SH |
| R3 | 15 | Cy5-AGTACCGTGAGGGAA- (CH$_2$)$_3$-SH |

DNAs (probe, target, reporter DNA) used in this experiment were purified with 1M DTT (dithiothreitol) and NAP-5-column (GE healthcare Co.). Au nanowire was incubated in 1 M KH$_2$P0$_4$ buffer (pH 6.75) containing 5 $\mu$M probe DNA at room temperature for 24 hours, followed by washing with 0.2%(w/v) SDS (sodium dodecyl sulfate) solution and drying in the presence of N$_2$ flowing.

Au nanowire on which probe DNA had been formed was transferred onto Si single crystal board on which M-PEG silane (methoxy-polyethylene glycol Silane) was formed by using nanomanipulator.

Au nanoparticle was modified by the reporter DNA labeled with Raman dye. Particularly, 0.01 M PB (phosphate buffer) solution containing 5 $\mu$M reporter DNA and 0.01%(w/v) SDS (sodium dodecyl sulfate) was mixed with Au nanoparticle, followed by incubation at room temperature for 12 hours. 2 M NaC1 was added to increase NaC1 concentration in PB solution up to 0.7 M (salt aging). After salt aging, Au nanoparticle was recovered by centrifugation, and the recovered nanoparticle was washed with 0.01%(w/v) SDS twice, followed by dispersion in PBS (phosphate buffered saline) solution.

Target DNA was added to hybridization buffer. The hybridization buffer herein was the mixed solution of 6x SSPE (0.9 M NaC1, 10 mM NaH$_2$PO$_4$H$_2$O, 1 mM EDTA, pH 7. 4) , 20% (v/v) formamide solution (Sigma-Aldrich) and 0.1% (w/v) SDS (sodium dodecyl sulfate).

Probe DNA attached Au nanowire was dipped in the hybridization buffer containing target DNA, followed by hybridization at 30°C for 6 hours and washing with 2x SSPE.

After the hybridization of probe DNA conjugated Au nanowire and target DNA, the hybridized Au nanowire was dipped in PBS (phosphate buffered saline) solution containing 0.l%(w/v) SDS in which reporter DNA conjugated Au nanoparticles were dispersed, followed by further hybridization for 6 hours.

After the hybridization of the hybridized Au nanowire and Au nanoparticle, the mixture was washed with PBS (phosphate buffered saline) containing 0.1% (w/v) SDS and deionized water, followed by drying in the presence of N$_2$ flowing. As a result, the DNA detection sample in which Au nanoparticles were self-assembled on Au nanowire having the target DNA in the middle of them was prepared.

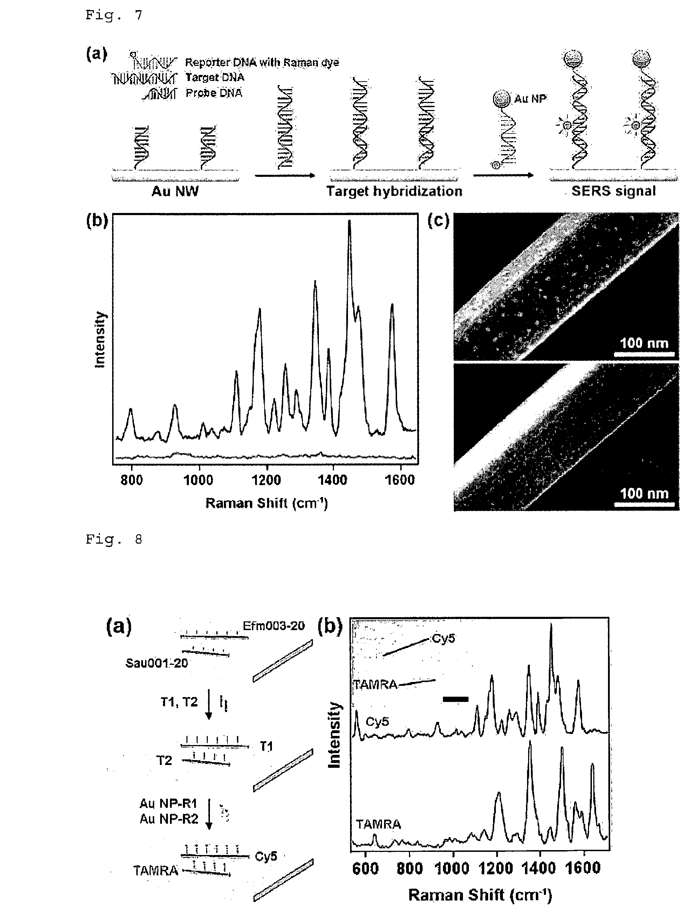

Figure 7(a) is a diagram illustrating an example of DNA detection using target DNA, reporter DNA, and probe DNA. As shown in Figure 7(a), hot spot and SERS signal can be obtained only by molecular recognition through DNA complementary binding.

Figure 7(b) and Figure 7(c) show the results of DNA detection using Efm003-20 probe DNA, T1 target DNA, T2 non-specific target DNA, and R1 reporter DNA.

The blue Raman spectrum shown in Figure 7(b) indicates the result of DNA detection obtained by hybridization using complementary binding target DNA. The purple Raman spectrum shown in Figure 7(b) indicates the result obtained by hybridization similar to the above but using non-complementary DNA instead of the target DNA above. As shown in Figure 7(b), only when target DNA was presented, strong SERS signal of Cy5 was obtained. When non-complementary DNA was used, weak Si band alone was observed.

The single nanowire itself can be SERS-active material, but when nanoparticles are attached on the surface, much stronger SERS signals can be obtained. Compared with the single nanowire, the experimental SERS enhancement factor of Au nanoparticle-Au nanowire structure having the target in the middle between them was 2.6 x 10$^3$.

Figure 7(c) is a set of SEM photographs for the direct observation of Au nanoparticle-Au nanowire structure resulted from DNA complementary binding. Precisely, typical Au nanoparticle-Au nanowire structure was formed by the hybridization with complementary target DNA (upper part of Figure 7(c)). However, when non-specific DNA was used, only nanowire was presented because nanoparticles were not formed (lower part of Figure 7(c)).

Figure 7(c) is a set of SEM photographs corresponding to SERS spectrum of Figure 7(b). In this Figure, the gap formation between nanoparticle-nanowire and the characteristic structure in which a biochemical material, the detection target, was fixed in the gap were confirmed to be very important factors for SERS enhancement. It also draws our attention that nanoparticles are evenly distributed on nanowire without being aggregated, just like avidin. Such even nanoparticle-nanowire structure is extremely well-defined structure, so that it can provide reproducible SERS signals.

For multiplex DNA detection, two Au nanowires modified with different probe DNAs (Efm003-20 and Sau001-20) were used. Those two Au nanowires modified with Efm003-20 and Sau001-20 were located on the same Si board.

As shown in Figure 8(a), this board containing Au nanowire modified with probe DNA was reacted with the target DNA mixture (T1 and T2) and Au nanoparticle mixture modified with reporter DNAs (R1 and R2) stepwise.

Independent SERS spectrum was measured from the resultant two Au nanoparticle-Au nanowire structures because one of those target DNAs (T1) was designed to be complementary to Efm003-20 and Cy5 reporter molecule (R1) and the other target DNA (T2) was designed to be complementary to SauOO1-20 and TAMRA reporter molecule (R2).

Figure 8(b) illustrates SERS spectrum obtained from each Au nanoparticle-Au nanowire structure. In this Figure, Cy5 Raman band was observed from the said Au nanoparticle-Au nanowire structure, which supported the idea that only T1 and R1 were presented in the system. Likewise, TAMRA SERS signals were obtained from the below Au nanoparticle-Au nanowire, suggesting that only T2 and R2 were presented therein.

The above results indicate that cross hybridization does not occur among different target DNAs. In other words, multiplex DNA detection is possible with one nanowire acting as one target DNA detection system.

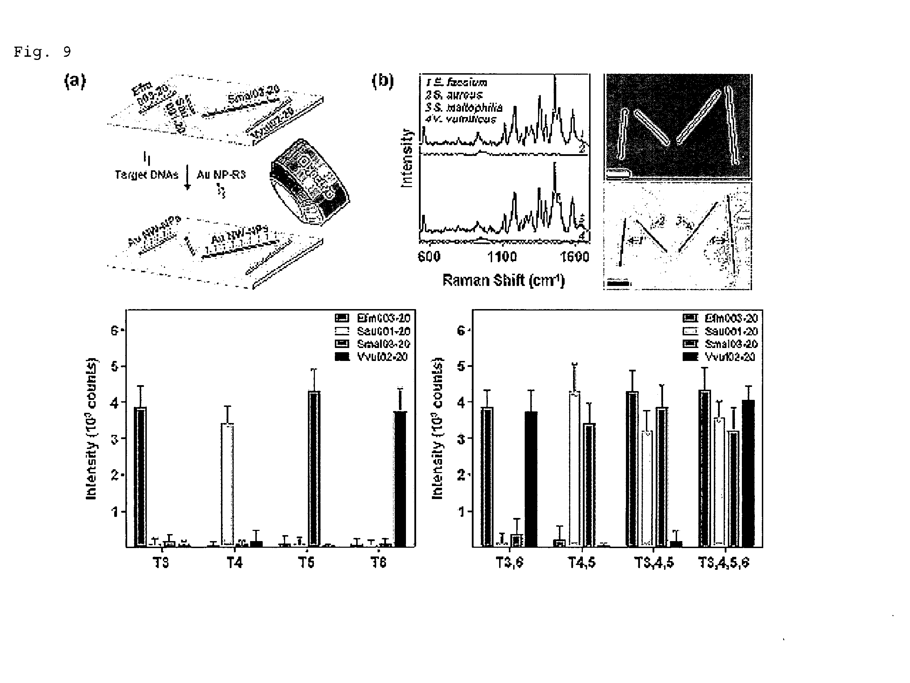

Figure 9 is another example of DNA detection using 4 Au nanowires. As shown in Figure 9(a), 4 Au nanowires each conjugated with different probe DNAs (Efm003-20, Sau1001-20, Sma103-20, and Wu102-30) were located on the same Si board following the shape of letter M. Each nanowire was classified by location address based on the shape of letter M.

It is very important to place each nanowire on the exact spot for the nanowire based multiplex detection. The single Au nanowire of the present invention can be transferred by nanomanipulator and can be observed easily under optical microscope. So, its location can be easily controlled and thus any additional patterning process is not required for the distinguishment of each nanowire.

The 4 kinds of nanowires on the board were mixed with different target DNAs (T3, T4, T5, and T6), and then 5'-termini were incubated with Cy5 and 3'-termini were incubated with the reporter DNA (R3) conjugated Au nanoparticle.

In this experiment, the length of the reporter DNA was limited to 15-mer to increase accuracy of the test. Short oligonucleotides could exclude cross reaction with other DNAs and rather increase discriminatory power.

Figure 9(b) - Figure 9(d) illustrate multiplex DNA detection using SERS of 4 platform samples composed of 4 kinds of Au nanowires. Those Au nanowires each having different probe DNA formed the shape of letter M on Si board, as shown in the optical microscope photograph of Figure 9(b). Each nanowire could be classified from the fixed location of Au nanowire and different target DNAs could be detected by one time analysis.

Figure 9(b) illustrates SERS spectra obtained from 4 different nanowires resulted from the experiment using the mixture of 2 target DNAs (T3 and T5). Only those nanowires conjugated with Efm003-20 and Sma103-20 displayed Cy5 SERS spectra.

Details of evaluation of this method are illustrated in Figure 9(c) and Figure 9(d). In those figures, the size of 1580 cm$^{-1}$ Raman band obtained from each Au nanowire of the board after the experiment with the mixture of different target DNAs was showed. As shown in Figure 9(c), SERS signal was observed in only one kind of Au nanowire having the probe DNA complementary to the target DNA because one of 4 target DNAs was used. As shown in Figure 9(d), sensing power for the multiplex DNA detection was clearly confirmed. When the mixture of T3 and T6 was loaded on the board, SERS signal was obtained only from the nanowire attached with Efm003-20 or Vvul02-20. When the mixture of T4 and T5 was used, only the nanowire attached with Sau001-20 or Sma103-20 displayed strong Cy5 SERS spectra. When the mixture of T3, T4, and T5 was used, all the nanowires except the one modified with Vvu102-20 displayed strong SERS signals. Lastly, the present inventors observed that all the Au nanowires on the chip displayed similar SERS spectra when the mixture of 4 kinds of target DNAs was used. This result indicates that Au nanowire-nanoparticle system facilitates the detection of sequence-specific DNA binding.

It is an important fact that the explained multiplexing capacity of platform is not attributed to different Raman dyes. In this experiment, only one reporter DNA was used for the multiplex DNA detection compared with another nanoparticle based detection method.

Even though SERS spectra of different dyes could be distinguished from each other because of narrow Raman band, it could be more advantageous to use the same Raman dye for quantitative multiplex detection. When different dyes are used for multiplex DNA detection, SERS enhancement can be varied from dyes.

Because it is easy to observe and transfer the single nanowire, the single crystal Au nanowire without any physically marked label such as barcode pattern can be used. Those Au nanowires that are not distinguished optically can be classified by the specific location on the board. Moreover, if the single crystal Au nanowire having a very smooth surface is used as a building block of SERS platform applicable for multiplex detection, SERS spectra having reproducibility that is one of the most important properties required for the detection by using SERS can be obtained.

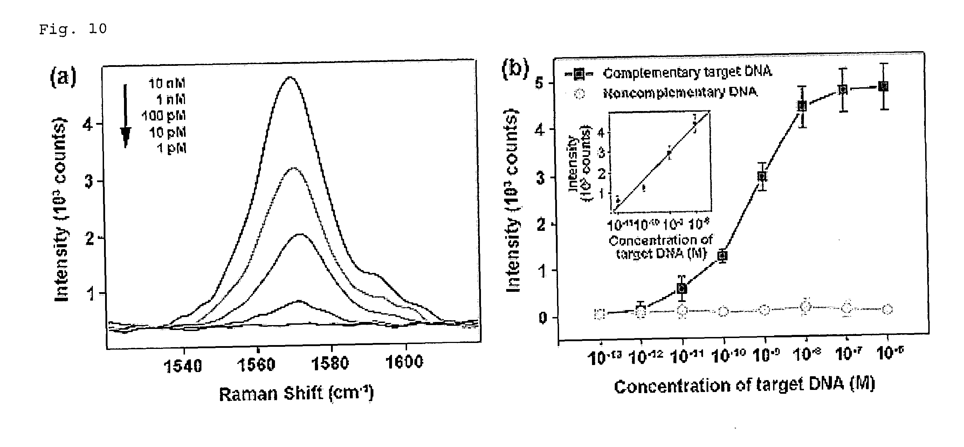

Figure 10 illustrates the result of investigation of the relation between the sensitivity of Au nanowire-Au nanoparticle structure and the concentration of target DNA (mols). The measurement was performed at different target DNA concentrations (at different mols). Figure 10(a) shows the changes of 1580 cm$^{-1}$ Raman band size of Cy5 over the changes of target DNA concentration. Herein, SERS signal was reduced in proportion to the concentration of target DNA, but still observed even at the low concentration of 10 pM.

Details of the investigation of the relation between the SERS size and the DNA concentration are illustrated in Figure 10(b). In Figure 10(b), the blue line indicates that the SERS size of Au nanowire-Au nanoparticle structure is closely related to the target DNA concentration. The limit of detection herein was presumed to be 10 pM, which was consistent with 300 amol (1.8 x 10$^8$ molecules) at the general assay volume 30 $\mu$l. SERS signal was saturated at 10 nM and had dynamic range of 3 orders.

Correlation of the SERS size and the concentration (correlation coefficient: 0.987) was confirmed in the 3 orders of dynamic range of 10$^{-11}$ - 10$^{-8}$. This result suggests that the quantitative detection of target DNA could be possible by using the Au nanowire-Au nanoparticle structure. In the comparative experiment using non-specific DNA, distinguishable SERS signal was not observed (Figure 10(b), purple line).

Detection of pathogenic DNA

It is very important clinically to detect a pathogen because such pathogen is responsible for numbers of contagious diseases having high disease rate and high death rate. Enterococcus faecium (E. faecium) and Staphylococcus aureus (S. aureus) are two most important pathogens causing vascular disease having high incidence rate and death rate. Stenotrophomonas maltophilia (S. maltophilia) is a highly risky pathogen for those patients with weak immune system, with organ transplantation, and with cystic fibrosis (CF). Vibrio vulnificus (V. vulnificus) proliferates very fast, taking only a few days to grow, to cause sepsis bearing high death rate of at least 50% and gastroenteritis.

Table 2 shows the summary of pathogens used for DNA detection.

(Table 2)

| Species | Source |
|---|---|
| *E. faecium* | KCCM[a] 12118 |
| *S. aureus* | KCTC[b] 1621 |
| *S. maltophilia* | ATCC[c] 13637 |
| *V. vulnificus* | KCTC[b] 2962 |

[a]KCCM (Korea Culture Center of Microorganisms, Seoul, Korea), [b]KCTC (Korean Collection for Type Cultures, Daejeon, Korea), [c]ATCC (American Type Culture Collection , Rockville, MD, USA)

Reference DNAs were isolated from the pathogens of Table 2 by using DNA mini kit (QIAGEN, Hilden, Germany) according to the manufacturer's protocol.

DNA of clinic pathogen was isolated from many clinical samples such as cerebrospinal fluid, feces, pus, and sputum taken from the patient infected with the pathogen by using QIAamp DNA Blood Mini Kit (QIAGEN) according to the manufacturer's protocol.

Species-specific DNA alone was separated from the isolated DNA and selectively amplified by PCR (polymerase chain reaction), which was then used as target DNAs. Particularly, PCR was performed using the reaction mixture comprising 1x Taq buffer, 0.2 mM dNTPs, 2 units of *Taq* polymerase (Takara Shuzo Co., Shiga, Japan), 5 ng genomic DNA, 5 pmol forward primer, and 25 pmol reverse prime.

For the PCR amplification, Efm003-20 (for E. *faecium),* Sau001-20 *(S. aureus),* Sma103-20 *(S. maltophilia)* or Vvu102-20 *(V. vulnificus)* was used as the species-specific primer, and 23BR (5'-TTCGCCTTTCCCTCACGGTACT-3') was used as the universal primer.

The amplified PCR product was separated and purified by using PCR purification kit (iNtRON Co., Ltd, Gyeongido, Korea) according to the manufacturer's protocol.

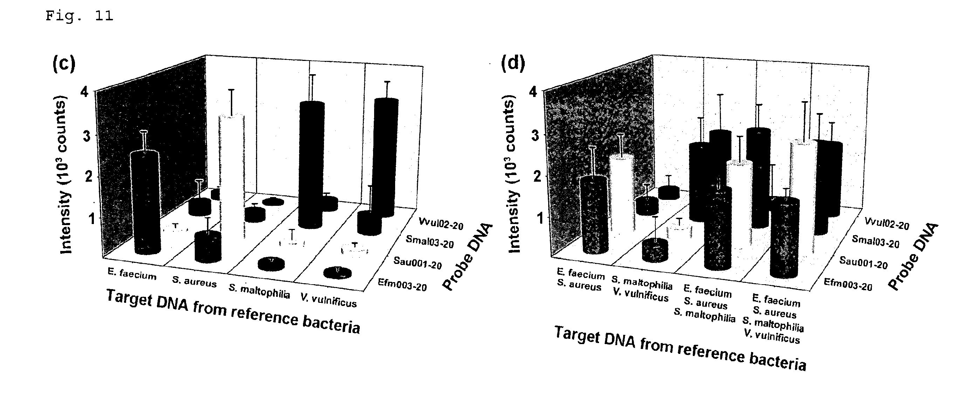

Figure 11 shows the result of SERS detection from multiplex pathogenic bacteria DNA (reference bacteria DNA) by using Au nanowire-Au nanoparticle structure.

SERS signal was amplified only in Au nanowire having the probe DNA complementary to the bacteria DNA. Even though this target DNA was much longer than the synthesized 35-mer oligonucleotides used for model system (S. maltophilia: 119bp; V. vulnificus: 131 bp; S. aureus: 192 bp; and E. faecium: 347 bp), the SERS detection result was similar to that of the model system. Detection of label-free DNA using Au nanowire-Au nanoparticle structure was not affected by the length of target DNA. That was because the distance between nanowire and nanoparticle could be reduced to small enough for the enhancement of SERS by DNA collapse under the dry condition.

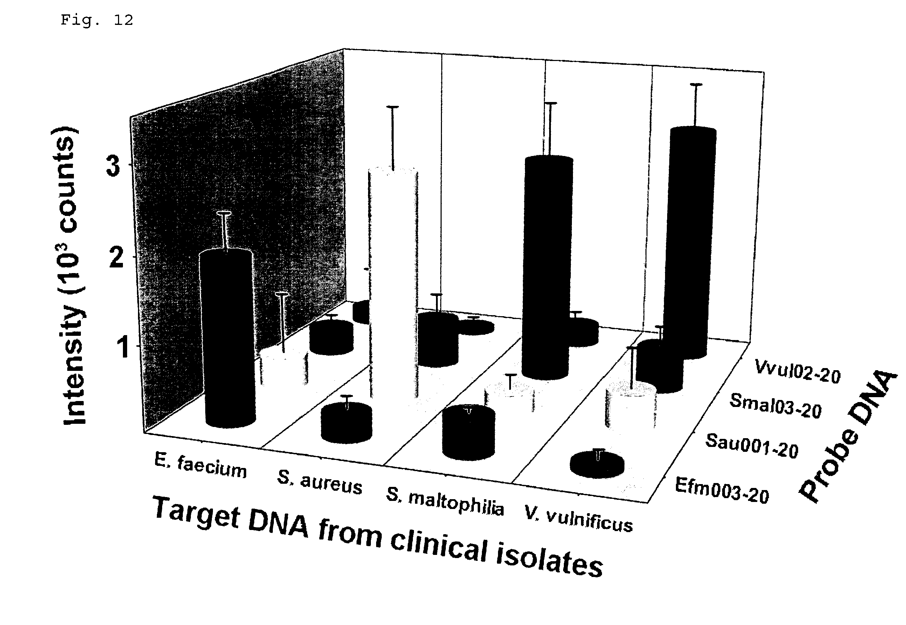

Figure 12 illustrates the result of SERS detection using Au nanowire-Au nanoparticle structure, for which clinic pathogen DNA was amplified by PCR and used as the target DNA. The result of clinic pathogen DNA detection shown in Figure 12 is similar with the result of reference bacteria DNA detection shown in Figure 11, suggesting that it is possible to detect a pathogen of an infected patient from SERS signal.

Four kinds of pathogens were successfully detected from 7 clinical samples by using Au nanowire-Au nanoparticle structure (E. faecium: 2, S. aureus: 2, S. maltophilia :2, and V. vulnificus: 1). The detection result obtained by using SERS was consistent with that of the conventional culture-based assay.

The detection method of a biochemical material of the present invention does not require pre-treatment of the biochemical material and is rather characterized by direct detection of the biochemical material. Even though a biochemical material, the detection target, was not labeled artificially with a marker, it could be selectively detected, that is so called non-labeled selective detection. In addition, a target biochemical material was fixed in hot spot region, so that even an extremely small amount of target material could be detected, suggesting that the method of the invention was high sensitive detection method. According to the present invention, physically separated each nanoparticle was self-assembled evenly on the surface of single crystal noble metal nanowire having a significantly big aspect ratio, resulting in nanowire-nanoparticle structure that had advantageous for giving a very stable and reproducible SERS spectrum. Besides, the single noble metal nanowire and the nanoparticles self assembled on the noble metal nanowire work together as a single sensor, providing high sensitivity, stability, and reproducibility along with possibility of microminiaturization of a sensor. The detection method of the present invention has other advantages as follows; location and density of hot spot can be regulated and SERS spectrum that is remarkably enhanced by the regulated multiple hot spots can be obtained. It is another advantage of the detection method of the present invention that multiplex platform of different detection targets is realized by the fast and single measurement with high accuracy and sensitivity resulted from forming receptors on multiple noble metal nanowires to be bound with different biochemical materials on each of them.

While the present invention has been described with respect to the specific embodiments, it will be apparent to those skilled in the art that various changes and modifications may be made without departing from the spirit and scope of the invention as defined in the following claims.

**Claims**

1.  A detection method of a biochemical material using surface-enhanced Raman scattering (SERS) in order to detect the existence of a biochemical material in a target subject or its content, comprising:

    contacting a detection target with single crystal noble metal nanowire having the first receptor formed thereon and with noble metal nanoparticles having the second receptor formed thereon in order to contact the detection target with the first receptor and with the second receptor, and accordingly binding the noble metal nanoparticles to the noble metal nanowire having the detection target in the middle, and
    obtaining SERS spectrum by irradiating polarized laser beam on the noble metal nanowire conjugated with the noble metal nanoparticles, mainly focusing on the nanowire.

2.  The detection method of a biochemical material according to claim 1, wherein the binding of the detection target with the noble metal nanowire or with the noble metal nanoparticle is performed by the bond between enzyme-substrate, antigen-antibody, protein-protein, biotin-avidin or the complementary bond between DNAs.

3.  The detection method of a biochemical material according to claim 1, wherein the length of long axis of the noble metal nanowire is at least 1 $\mu$m and the aspect ratio (nanowire long axis length/short axis length) of the noble metal nanowire is 5 - 150.

4.  The detection method of a biochemical material according to claim 3, wherein the mean diameter of the noble metal nanoparticle is 5 nm - 20 nm.

5.  The detection method of a biochemical material according to claim 1, wherein the joint density that is the number of the noble metal nanoparticle conjugated on the surface of the noble metal nanowire per unit area is the same as hot spot density that is the number of hot spots located on the surface of the noble metal nanowire per unit area.

6.  The detection method of a biochemical material according to claim 2, wherein the detection target includes avidin, the first receptor and the second receptor include biotin respectively, and the noble metal nanoparticles are self-assembled on the surface of the noble metal nanowire via biotin-avidin-biotin bond which is the bond of biotins respectively formed on the noble metal nanowire and the noble metal nanoparticle with having avidin in the middle.

7.  The detection method of a biochemical material according to claim 6, wherein the avidin is specifically bound with the biochemical material, the detection target.

8. The detection method of a biochemical material according to claim 2, wherein the detection target includes target DNA, the first receptor includes probe DNA, the second receptor includes Raman dye conjugated reporter DNA, and the noble metal nanoparticles are self-assembled on the surface of the noble metal nanowire via complementary bond between the target DNA and the probe DNA and complementary bond between the target DNA and the reporter DNA.

9. The detection method of a biochemical material according to claim 1, wherein the detection target is contacted with two or more single crystal noble metal nanowires on which different first receptors are formed with physically separated each other and with twp or more noble metal nanoparticles on which different second receptors are formed with physically separated each other, so as to detect different biochemical materials from each noble metal nanowire.

10. The detection method of a biochemical material according to claim 1, wherein the detection target containing 1 - N numbers of target DNA is contacted with at least N numbers (N is a natural number bigger than 1, N>1) of the single crystal noble metal nanowires on which different probe DNAs have been formed and the single noble metal nanoparticle on which Raman dye conjugated reporter DNA has been formed, in order to detect different target DNAs from each noble metal nanowire.

11. The detection method of a biochemical material according to claim 9, wherein the said one or more noble metal nanowires are identified by location addressing on the board.

12. The detection method of a biochemical material according to claim 1, wherein the biochemical material, the detection target, is DNA, and the DNA concentration (M) is linearly in proportion with the strength of the SERS spectrum of step b), particularly at the concentration of $10^{-11}$ - $10^{-8}$.

13. The detection method of a biochemical material according to claim 3, wherein the noble metal nanowire is Au, Ag, Pt or Pd nanowire and the noble metal nanoparticle is the same Au, Ag, Pt, or PD nanoparticle as those for the noble metal nanowire.

14. The detection method of a biochemical material according to claim 1, wherein the SERS is generated by irradiating polarized laser beam focusing on the center of the long axis direction of the single noble metal nanowire.

Fig. 1

Fig. 2

Fig. 3

Fluid containing Bio-Materials

Fig. 4

Fig. 5

Fig. 6

(a)

(c)

Fig. 7

**(a)** Reporter DNA with Raman dye / Target DNA / Probe DNA

Au NW → Target hybridization → Au NP → SERS signal

**(b)** Intensity vs. Raman Shift (cm⁻¹): 800, 1000, 1200, 1400, 1600

**(c)** 100 nm / 100 nm

Fig. 8

**(a)** Efm003-20 / Sau001-20 / T1, T2 / T1 / T2 / Au NP-R1, Au NP-R2 / Cy5 / TAMRA

**(b)** Cy5 / TAMRA / Cy5 / TAMRA — Intensity vs. Raman Shift (cm⁻¹): 600, 800, 1000, 1200, 1400, 1600

Fig. 9

Fig. 10

Fig. 11

Fig. 12

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- KR 1020090096643
- US 6974669 B
- KR 0892629
- KR 1020070065030
- KR 1020080036360
- KR 1020090035533

### Non-patent literature cited in the description

- **CHOU et al.** *Biosens. Bioelectron.,* 2004, vol. 19, 999-1005
- **ALIVISATOS et al.** *Nat. Biotechnol.,* 2004, vol. 22, 47-52
- **NAM et al.** *Science,* 2003, vol. 301, 1884-1886
- **TAO et al.** *Nano. Lett.,* 2003, vol. 3, 1229
- **JEONG et al.** *J. Phys. Chem. B,* 2004, vol. 108, 12724
- **AROCA et al.** *Anal. Chem.,* 2005, vol. 77, 378
- **SCHNEIDER et al.** *J. Appl. Phys.,* 2005, vol. 97, 024308
- **LEE et al.** *J. Am. Chem. Soc.,* 2006, vol. 128, 2200
- **PROKE et al.** *Appl. Phys. Lett.,* 2007, vol. 90, 093105

SEQUENCE LISTING

<110>     KOREA ADVANCED INSTITUTE OF SCIENCE AND TECHNOLOGY

<120>     DETECTION METHOD OF BIO-CHEMICAL MATERIAL USING SURFACE-ENHANCED RAMAN
          SCATTERING

<130>     21983EP

<140>     10 187 095.4
<141>     2010-10-11

<150>     KR 10-2009-0096643
<151>     2009-10-12

<160>     9

<170>     KopatentIn 1.71

<210>     1
<211>     40
<212>     DNA
<213>     Artificial Sequence

<220>
<223>     T1


<400>     1
agtaccgtga gggaaaggcg ctacctcgaa tgtgctatgt                              40


<210>     2
<211>     40
<212>     DNA
<213>     Artificial Sequence

<220>
<223>     T2


<400>     2
tgttacgatt gtgtgaatac tcgtctaatg tcgtcctttg                              40


<210>     3
<211>     35
<212>     DNA
<213>     Artificial Sequence

<220>
<223>     T3


<400>     3
ttccctcacg gtactctacc tcgaatgtgc tatgt                                   35

```
<210>    4
<211>    35
<212>    DNA
<213>    Artificial Sequence

<220>
<223>    T4


<400>    4
ttccctcacg gtacttcgtc taatgtcgtc ctttg                              35


<210>    5
<211>    35
<212>    DNA
<213>    Artificial Sequence

<220>
<223>    T5


<400>    5
ttccctcacg gtactctcgt cttcactgga atggc                              35


<210>    6
<211>    35
<212>    DNA
<213>    Artificial Sequence

<220>
<223>    T6


<400>    6
ttccctcacg gtactgaaca tgcatcgtca actac                              35


<210>    7
<211>    20
<212>    DNA
<213>    Artificial Sequence

<220>
<223>    R1


<400>    7
cgcctttccc tcacggtact                                               20


<210>    8
<211>    20
<212>    DNA
<213>    Artificial Sequence
```

<220>
<223>    R2

<400>    8
gtattcacac aatcgtaaca                                                        20

<210>    9
<211>    15
<212>    DNA
<213>    Artificial Sequence

<220>
<223>    R3

<400>    9
agtaccgtga gggaa                                                             15